# FASCICULE DE BREVET EUROPEEN

(11) **EP 0 683 791 B1**
(45) Date de publication et mention de la délivrance du brevet: **07.04.2004**
(21) Numéro de dépôt: 94906253.3
(22) Date de dépôt: 09.02.1994
(51) Int. Cl.: C07K 1/14, C07K 14/435, A61K 38/00, G01N 33/569

(54) **COMPOSITION AQUEUSE PROTEINIQUE, GLYCOPROTEINE CONTENUE, PROCEDE D'OBTENTION ET UTILISATIONS**
WÄSSRIGE PROTEINZUSAMMENSETZUNG, ENTHALTENDE GLYKOPROTEINE, VERFAHREN ZUR HERSTELLUNG UND VERWENDUNGEN
AQUEOUS PROTEIN COMPOSITION, GLYCOPROTEIN CONTAINED THEREIN, PREPRATION METHOD THEREFOR AND USES THEREOF

(30) Priorité: 09.02.1993 FR 9301399
(43) Date de publication de la demande: 29.11.1995
(73) Titulaire: Apoh-Technologies SA, 34032 Montpellier (FR)
(72) Inventeur: Stefas, Elie, 34280 La Grande-Motte (FR); Rucheton, Marcel, 34000 Montpellier (FR); Graafland, Hubert, 34000 Montpellier (FR)
(74) Mandataire: Peuscet, Jacques
(86) Numéro de dépôt international: PCT/FR1994/000143
(87) Numéro de publication internationale: WO 1994/018228

(56) Documents cités:
- FR-A- 2 541 593
- CHEMICAL ABSTRACTS, vol. 89, no. 19, 6 Novembre 1978, Columbus, Ohio, US; abstract no. 159658, M. BURSTEIN ET AL. 'POLYSULFATES, ANIONIC DETERGENTS, SODIUM PHOSPHOTUNGSTATE AND THE ELECTROPHORETIC MOBILITY OF PLASMA PROTEINS.' page 256 ; & NOUV. REV. FR. HEMATOL./BLOOD CELLS vol. 20, no. 1 , 1978 pages 49 - 62
- PROCEEDINGS OF THE NATIONAL ACADEMY OF SCIENCES OF USA. vol. 81 , Juin 1984 , WASHINGTON US pages 3640 - 3644 J. LOZIER ET AL. 'COMPLETE AMINO ACID SEQUENCE OF HUMAN PLASMA BETA 2- GLYCOPROTEIN I.' cité dans la demande

## Description

La présente invention concerne une composition protéinique obtenue comme produit secondaire dans la production d'albumine à partir de plasma sanguin humain, son procédé d'obtention, la glycoprotéine qu'elle contient et l'utilisation de ladite glycoprotéine dans des agents de stabilisation de l'albumine et comme agent permettant la détection et/ou le dosage d'anticorps.

On sait que, dans l'état de la technique, on effectue, pour obtenir des solutions d'albumine humaine, un fractionnement du plasma sanguin soit par la méthode de COHN (voir Cohn J. et Al, J. Amer. Chem. Soc, 72, 465-474-(1950)), soit par une méthode dérivée dans laquelle on effectue des traitements thermiques et éthanoliques successifs ou une méthode analogue dans laquelle on utilise un agent de précipitation autre que l'éthanol (notamment éther, sulfate d'ammonium, polyéthylène glycol ou acide caprylique). Selon la méthode de Cohn, qui est, en pratique, la plus utilisée, le plasma est refroidi à - 30° C puis réchauffé à - 2° C d'où il résulte un cryoprécipité contenant le facteur VIII antihémophilique, le fibrinogène et la fibronectine. Le surnageant, dit "surnageant I", est séparé du précipité susmentionné ; on abaisse son pH à 5,85 ± 0,05 et on ajoute de l'éthanol jusqu'à ce que la concentration éthanolique soit de 19 % en volume, la température étant abaissée, au fur et à mesure de l'addition d'éthanol, jusqu'à - 5° C. On obtient ainsi un précipité, dit "précipité (II + III)", contenant notamment les gamma-globulines et un surnageant, dit "surnageant (II + III)" contenant l'albumine et des impuretés. On reprend le surnageant ainsi obtenu et on augmente le taux d'alcool jusqu'à obtenir une concentration éthanolique de 40 % en volume, la température étant abaissée à environ - 8° C. On obtient ainsi un précipité, dit "précipité IV", et un surnageant, dit "surnageant IV", qui contient l'albumine à un degré de pureté d'environ 94 à 97 % en poids. Le surnageant IV sert de matière première pour les solutions d'albumine désirées, mais il contient une grande quantité d'éthanol ; selon une première technique, on peut dialyser directement le surnageant IV contre du sérum physiologique mais il faut alors utiliser une très grande quantité d'eau et le procédé est donc lent et coûteux ; selon une autre technique, on abaisse le pH du surnageant IV à 4,80 ± 0,05 ce qui fait précipiter l'albumine : on sépare ce précipité, dit "précipité V", et on le remet en solution dans du sérum physiologique, le reste de l'éthanol étant extrait par dialyse.

Les solutions aqueuses obtenues après dialyse du "surnageant IV" ou du "précipité V" remis en solution contiennent de l'albumine et environ 4 % en poids, par rapport au poids total de matières protéiniques, d'autres protéines dites secondaires ou contaminantes et/ou de polymères.

Selon FR-A-2 690 444, on sépare l'albumine des autres protéines par un procédé chromatographique en phase liquide dans lequel on fait passer après dialyse la solution aqueuse contenant l'albumine dans au moins une colonne de chromatographie dite "hydrophobe" remplie d'un matériau particulaire apte à retenir une partie des protéines autres que l'albumine ; pour compléter la séparation, on propose également de faire passer la solution aqueuse d'albumine dans au moins une colonne de chromatographie d'affinité contenant un support particulaire neutre ou voisin de la neutralité chargé d'un composé polysulfaté. L'effluent obtenu par ce procédé est constitué par une solution d'albumine purifiée, la majeure partie des protéines secondaires étant fixées, soit sur la (les) colonne(s) de chromatographie hydrophobe, soit sur la (les) colonne(s) de chromatographie d'affinité.

Selon un mode préféré de mise en oeuvre de ce procédé, on soumet à la chromatographie une solution aqueuse d'albumine à un pH compris entre 6,5 et 7,8 et à une concentration supérieure à 1 g/l et inférieure à 400 g/l.

On choisit avantageusement, comme matériau particulaire apte à retenir les protéines contaminantes dans la chromatographie hydrophobe, un support particulaire neutre, dont les particules sont chargées d'un composé comportant des radicaux alkyle C₃-C₈ hydrophobes ; on peut choisir notamment, comme composé chargeant les particules du support, un composé comportant des radicaux butyle : le lit de remplissage est, par exemple, constitué du produit commercialisé par la société "MERCK" sous la dénomination commerciale "FRACTOGEL TSK BUTYL-650". La chromatographie d'affinité se fait, de préférence, sur gel de dextran sulfate. On choisit, de préférence, pour effectuer la chromatographie d'affinité et la chromatographie hydrophobe un matériau particulaire ayant une granulométrie de dimension moyenne inférieure à 300 µm. Le débit de chromatographie est généralement compris entre 10 et 20 cm/heure et la température entre 2 et 25 °C. La chromatographie d'affinité peut être effectuée après ou, de préférence, avant la chromatographie hydrophobe.

On a constaté, selon la présente invention, que l'on pouvait éluer les protéines secondaires fixées sur les lits de remplissage des colonnes de chromatographie susmentionnées et obtenir ainsi des compositions protéiniques utiles, notamment dans le domaine pharmaceutique.

La présente invention a, par conséquent, pour premier objet un procédé d'obtention d'une composition aqueuse protéinique à partir du plasma sanguin humain dans lequel on soumet le plasma à des traitements successifs par action de la température et par action d'un agent de précipitation pour aboutir, après séparation au moins partielle des facteurs VIII et IX et des gamma globulines, à des solutions d'albumine contenant l'agent de précipitation, puis on sépare, notamment par dialyse, l'agent de précipitation des solutions obtenues pour obtenir une solution aqueuse brute d'albumine et on soumet ladite solution aqueuse brute d'albumine à au moins une chromatographie en phase liquide pour retenir au moins une partie des protéines secondaires autres que l'albumine, ladite chromatographie en phase liquide comportant une chromatographie d'affinité sur un support particulaire constitué de particules chargées d'au moins un composé comportant des groupes sulfates, caractérisé par le fait qu'après passage de la solution d'albumine, on soumet le support particulaire de la chromatographie d'affinité précité à une élution et que l'on recueille en élution la composition protéinique désirée.

L'élution est, de préférence, effectuée en augmentant la force ionique par passage d'une solution saline. La solution saline est, de préférence, une solution de NaCl, à une concentration au moins égale à 0,3 M, avantageusement de 2 M ; elle est. avantageusement précédée d'une opération de lavage. Le lavage du support de chromatographie d'affinité est effectué, notamment, à l'aide d'un tampon salin, de molarité égale à 0,16 mole/l, de préférence un tampon phosphaté constitué de phosphates mono- et disodique, notamment à 0,01 mole/l, et de chlorure de sodium, notamment à 0,15 mole/l, dans des proportions donnant un pH de 7,00 ± 0,05. Le lavage est maintenu tant que la densité optique de l'effluent est supérieure à une valeur prédéterminée, par exemple 0,1 UDO (unité de densité optique).

La solution obtenue par élution de la (des) colonne(s) de chromatographie d'affinité contient un mélange de protéines. La présente invention a aussi pour objet la composition protéinique ainsi obtenue. Cette composition contient une glycoprotéine qui a un poids moléculaire de 50 000 ± 3 000 dalton après ou sans réduction par le 2-mercaptoéthanol. La glycoprotéine constitue de 5 à 100 % en poids du contenu protéinique total de la composition obtenue selon le mode de préparation ; généralement, l'élution permet d'obtenir une concentration pondérale comprise entre 0,05 g et 30 g de glycoprotéine par litre d'éluat mais cette concentration peut être réduite par dilution ou augmentée par concentration de l'éluat.

La glycoprotéine isolée selon l'invention présente les caractéristiques et propriétés données ci-après.

Les 20 premiers acides aminés de sa région N-terminale ont été déterminés en particulier par microséquençage en phase gazeuse à l'aide d'un appareil "Applied Biosystems Inc, modèle 470" couplé à un analyseur de phénylrhéohydantoïne modèle 120 A (ABI) :
Gly-Arg-Thr-Cys-Pro-Lys-Pro-Asp-Asp-Leu-Pro-Phe-Ser-Thre-Val-Val-Pro-Leu-Lys-Thre.
Cette séquence de 20 premiers acides aminés correspond à celle d'une β2-glycoprotéine plasmatique décrite (1) dans l'article de J. LOZIER et coll. Proc. Natl. Acad. Sci. ISA Vol. 81, pages 3640-3644, Juin 1984 et (2) dans l'article T. KRISTENSEN et coll. FEBS Letters, Vol. 289, 1991, pages 183-186.

La composition en acides aminés de la glycoprotéine isolée selon l'invention a été déterminée sur deux préparations différentes par deux laboratoires différents :
a) la première préparation A a été traitée par hydrolyse acide dans HCl 5,7 M pendant 24 heures à 110° C avec un analyseur d'acides aminés "BECKMAN 6 300" relié à un système "GOLD" pour interprétation,
b) la seconde prépration B a également été analysée avec un analyseur d'acides aminés "BECKMAN 6 300" après hydrolyse par HCl 6 N pendant 24 heures et 72 heures.

Les résultats obtenus sont donnés dans le tableau I ci-après, où ils sont comparés avec les résultats publiés par J. LOZIER et coll. (1) et T. KRISTENSEN et coll. (2). Ces résultats montrent que la glycoprotéine est une β2'-glycoprotéine I voisine de la β2-glycoprotéine I décrite par J. LOZIER et coll. et T. KRISTENSEN et coll.

Cependant, cette glycoprotéine diffère de la β2-glycoprotéine I. En effet, après purification, précipitation de la glycoprotéine selon l'invention à l'acétone et dialyse contre de l'eau distillée, le séquençage donne lieu à l'identification de 14 acides aminés, sortant 2 à 2, et correspondant à la région N-terminale de la protéine. Ceci correspond, étant donné le degré de pureté de la préparation protéique, au clivage de la protéine de départ. En effet, 7 de ces acides aminés correspondent à la région N-terminale. Il s'agit de la séquence :
Gly-Arg-Thr-Cys-Pro-Lys-Pro
Les autres 7 acides aminés, par déduction correspondent au peptide :
Phe-Trp-Lys-Ser-Asp-Ala-Ser
Ce peptide correspond à la région comprise entre les acides aminés 315 et 321 qui a été décrite par J. LOZIER pour la β2-glycoprotéine I mais une différence d'un acide aminé apparaît. Il s'agit d'une sérine à la place d'une thréonine. La glycoprotéine isolée selon l'invention est donc un allotype de la β2-glycoprotéine I que l'on appellera par la suite β2'-glycoprotéine I.

Après clivage de la protéine au bromure de cyanogène, séparation des peptides obtenus et analyse dans chaque peptide des trois premiers acides aminés, on retrouve les résultats décrits par J. LOZIER et coll.

La présente invention a donc pour second objet la composition protéinique obtenue par élution de la (des) colonne(s) de chromatographie d'affinité dans le procédé décrit ci-dessus et la β2'-glycoprotéine I, telle que définie ci-dessus.

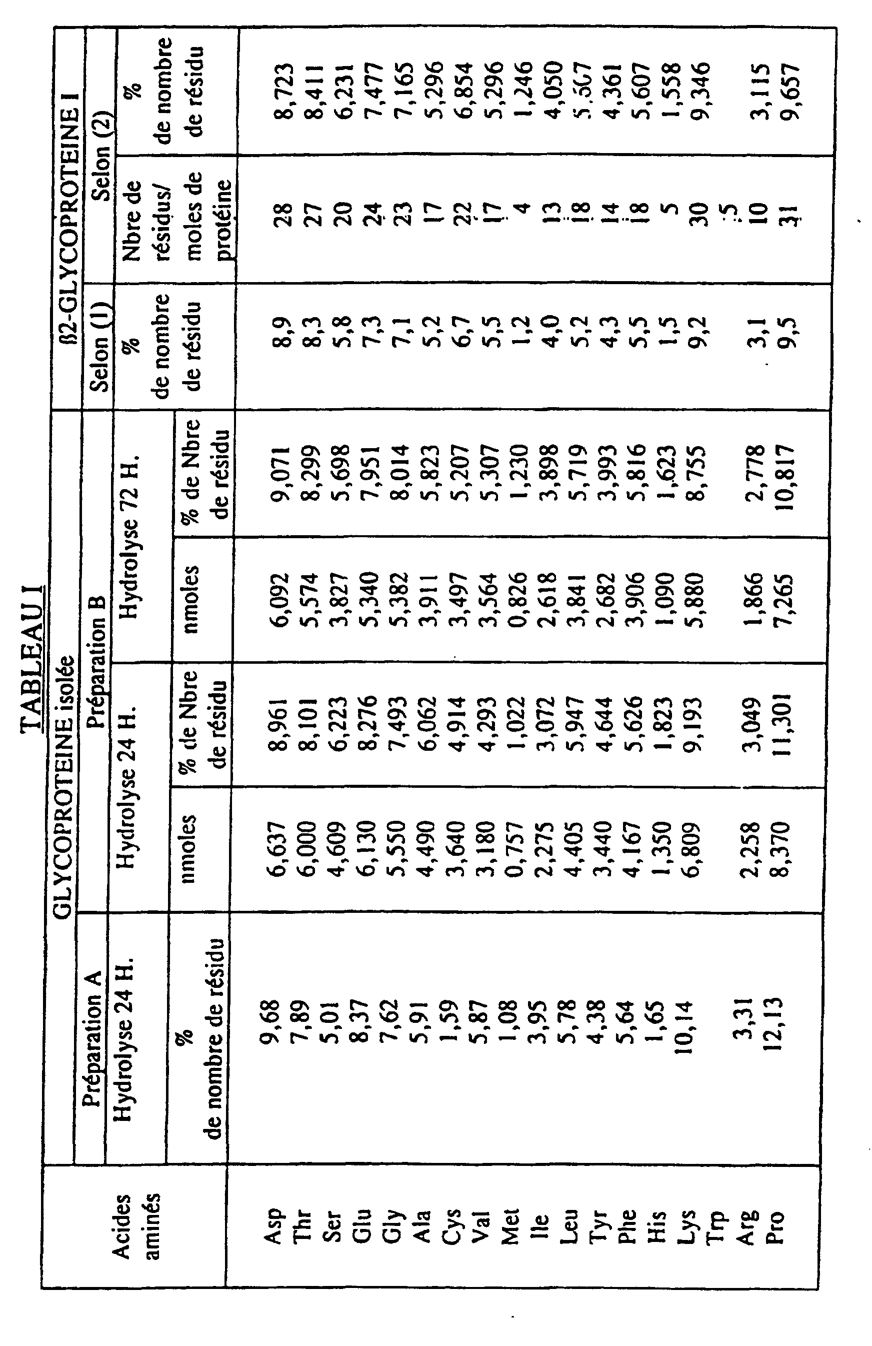

On a trouvé que la composition protéinique ainsi que la β2'-glycoprotéine sont antigéniques, c'est-à-dire détectées par la méthode ELISA ou par immuno-empreinte, par des anticorps de sujets humains, par exemple pour des sujets infectés par HBV, HIV, du syndrome de Gougerot-Sjögren ; elles sont également reconnues dans certaines conditions, en. ELISA, par des. anticorps monoclonaux spécifiques de la p 25/p 55 gag du virus HIV1 (Ac. Mo. RL4.72.1) et de la protéine antioncogène p 53 (Ac. Mo. 122) ; on peut donc utiliser la composition et la β2'-glycoprotéine I pour la caractérisation et le dosage des anticorps contre cette protéine chez des malades atteints de SIDA, d'hépatite B, de leucémies, du syndrome de Gougerot-Sjôgren, ou de myélomes. La méthode ELISA est, par exemple, décrite dans l'article de ENGVALL et coll. Immunochemistry 1971, 8, pages 871 à 879 et la méthode par immuno-empreinte est décrite, par exemple, dans TOWBIN et coll Proc. Natl. Acad. Sci. USA, 1979, 76, pages 4350-4354.

La composition protéinique et la β2'-glycoprotéine I inhibent la dénaturation de l'albumine à 60° C observée en turbidimétrie, de façon "dose dépendante" ; on peut donc l'utiliser pour stabiliser l'albumine, notamment lors de la tyndallisation.

La β2'-glycoprotéine I peut être séparée des protéines secondaires avec lesquelles elle est mélangée dans la composition protéinique obtenue par élution de la colonne de chromatographie d'affinité, de la façon décrite ci-après : la composition est éluée du gel de dextran sulfate par une solution de NaCl au moins égale à 0,3 M, avantageusement voisine de 2 moles par litre ; l'éluat obtenu est dilué dans un tampon salin de façon à abaisser sa force ionique à moins de 0,2 ; la solution obtenue est soumise à une chromatographie sur gel phosphaté, puis la glycoprotéine fixée sur le gel phosphaté est éluée en augmentant la force ionique avec un tampon salin ayant, de préférence, une force ionique supérieure à 0,4. Le débit de la chromatographie sur gel phosphaté est, de préférence, compris entre 10 et 20 cm/heure et la température est, de préférence, comprise entre 0 et 40° C, de préférence 2 et 25 °C, en conditions usuelles.

L'invention a également pour objet un agent de stabilisation de l'albumine lors de la tyndallisation contenant la composition protéinique ou la β2'-glycoprotéine I définie ci-dessus et un agent permettant la détection et/ou le dosage d'anticorps de sujets humains infectés par HBV, HIV, du syndrome de Gougerot-Sjögren ou de myélomes, par la méthode ELISA ou par Immuno-empreinte caractérisé par le fait qu'il contient la composition protéinique ou la β2'-glycoprotéine I définie ci-dessus.

Pour mieux faire comprendre l'invention, on va décrire ci-après, à titre d'exemple purement illustratif et non limitatif, un mode de préparation de la β2'-glycoprotéine I selon l'invention.

On utilise, comme matière première de départ, un plasma humain fractionné selon le procédé décrit par KISTLER et NITSCHMAN (Vox Sang. 7, 414-424 (1962)), qui est une méthode dérivée de celle de COHN, les fractions de départ contenant l'albumine étant soit le surnageant IV, soit le précipité V.

Lorsque l'on part du surnageant IV, qui comprend 40 % (en volume) d'éthanol et qui a un pH de 5,85 ± 0,05, on dilue de moitié le surnageant avec une solution de NaCl à 7 g/l. Le pH est ajusté à 7,45 ± 0,05 avec une solution de soude 1N. On préconcentre l'albumine jusqu'à 90 g/l dans une cassette d'ultrafiltration de type "Oméga" (Filtron) mise en oeuvre dans un système d'ultrafiltration "Minisette SS Cell NPT Cell" (Filtron Techn. Corp.). Cette cassette a une surface filtrante de 0,07 m² et un seuil de rétention de 30 KDa. La circulation est assurée par une pompe péristaltique à une pression qui varie de 2 x 10⁵ Pa au début de l'opération à 5 x 10⁵ Pa vers la fin. Lorsque l'on a atteint la concentration de 90 g/l en albumine, on ajoute à la solution d'albumine une solution de NaCl à 9 g/l et on continue à dialyser à volume constant jusqu'à obtenir un taux d'éthanol inférieur à 0,1 % en volume. Lorsque l'éthanol a été ainsi éliminé, on poursuit la dialyse en concentrant la solution jusqu'à 200 g d'albumine par litre. On ajuste le pH à 7,10 ± 0,05 avec une solution d'acide chlorhydrique 1N.

Lorsque l'on part du précipité V, on remet ledit précipité en suspension dans du sérum physiologique (solution de NaCl à 9 g par litre) en utilisant 4 litres de sérum physiologique par kilogramme de précipité. On filtre pour éliminer les grumeaux de précipité non remis en suspension au cours de l'agitation ; on ajuste le pH à 7,45 ± 0,05 avec une solution de soude 1N et on concentre la solution ainsi obtenue jusqu'à 90 g de protéines par litre en utilisant le même système de dialyse que précédemment décrit pour le surnageant IV. On poursuit alors la dialyse en rajoutant du sérum physiologique et en travaillant à volume constant jusqu'à obtenir un taux final d'éthanol inférieur à 0,1 % en volume. Lorsque l'éthanol a été ainsi éliminé, on poursuit la dialyse pour concentrer la solution jusqu'à environ 200 g de protéines par litre. On ajuste le pH à 7,10 ± 0,05 avec une solution d'acide chlorhydrique 1N.

La solution d'albumine ainsi préparée à partir du surnageant IV ou du précipité V est alors filtrée stérilement (filtre "Millex-GS" de 0,2 micron (Millipore)).

La solution aqueuse brute d'albumine obtenue subit d'abord une chromatographie d'affinité. Cette chromatographie est réalisée dans une colonne de 50 ml (2,5 cm x 10 cm) (Biorad), chargée avec un matériau particulaire vendu par la société "SIGMA" sous la dénomination commerciale "DEXTRAN BEADS SULFATED". La colonne est préalablement équilibrée en faisant passer dans le lit 3 volumes de colonne de sérum physiologique. La solution d'albumine est alors envoyée sur cette colonne et l'effluent peut être récupéré pour la production d'albumine.

Le déroulement de la chromatographie est suivi en mesurant la densité optique à 280 nm des fractions en sortie de colonne. Le débit de la colonne est de 16 cm/heure ; la chromatographie est effectuée entre 20 et 25 ° C.

Après passage de l'albumine récupérée comme effluent, la colonne d'affinité est lavée avec un tampon salin, voisin de l'isotonicité, de préférence un tampon constitué de phosphates mono- et disodique, notamment à 0,01 mole/l, et de chlorure de sodium, notamment à 0,15 mole/l ayant un pH de 7,00 ± 0,05 jusqu'à ce que la densité optique de l'effluent soit inférieure à 0,1. Les protéines fixées sur la colonne d'affinité sont alors éluées, en augmentant la force ionique, par passage d'une solution 2 M de NaCl. Le lavage et l'élution sont effectués à température ambiante, les débits de lavage et d'élution étant de 16 cm/h.

On a effectué, sur la solution obtenue, une électrophorèse dénaturante SDS-Page. On a constaté la présence d'une bande diffuse correspondant à la présence d'une protéine ayant un poids moléculaire de 50 000 ± 3 000 daltons après ou sans réduction par le 2-mercaptoéthanol. La technique de coloration Schiff montre que cette protéine est une glycoprotéine. Après coloration du gel en bleu de Coomassie, dans cette expérience, l'étude de la surface des pics de densité optique montre que la composition contient 55 % en poids de β2'-glycoprotéine I par rapport aux protéines totales.

La β2'-glycoprotéine I est ensuite séparée et purifiée de la façon suivante : la composition protéinique obtenue par élution de la (des) colonne(s) de chromatographie d'affinité à l'aide d'une solution saline de NaCl à 2 moles/litre, est diluée 10 fois dans un tampon constitué de phosphates mono- et disodique, notamment à 0,01 mole/litre, dans des proportions donnant un pH de 6,8 ± 0,05. Elle subit alors une chromatographie sur gel phosphaté. Cette chromatographie est réalisée dans une colonne de 50 ml (2,5 cm x 10 cm) (Biorad), chargée avec un matériau particulaire vendu par la société "BIO-RAD" sous la dénomination commerciale "BIO-GEL HTP". La colonne est préalablement équilibrée en faisant passer dans le lit 3 volumes de colonne de tampon constitué de phosphates mono- et disodique, notamment à 0,01 mole/litre, dans des proportions donnant un pH de 6,8 ± 0,05. L'éluat protéinique de la précédente chromatographie, dilué, est alors envoyé sur cette colonne. La chromatographie est effectuée avec un débit d'environ 15 cm/heure à une température de 20° C. L'effluent est éliminé et le support chromatographique subit ensuite un lavage effectué à l'aide d'un tampon phosphaté, identique à celui ayant servi à équilibrer la colonne. Le lavage est maintenu tant que la densité optique de l'effluent est supérieure à une valeur prédéterminée, par exemple 0,1 UDO (unité de densité optique).

La β2'-glycoprotéine I qui, dans ces conditions, se fixe sur le gel phosphaté, est alors éluée en augmentant la force ionique. L'opération d'élution est, de préférence, réalisée au moyen d'une solution de KCl ayant une concentration voisine de 1M. Cette solution d'élution est constituée de phosphates mono- et disodique, notamment à 0,01 mole/litre, dans des proportions donnant un pH de 6,8 ± 0,05 et de KCl 1 mole/litre. Le déroulement de la chromatographie est suivi en mesurant la densité optique à 280 nm des fractions en sortie de colonne. Le débit d'équilibrage de la colonne, de charge, de lavage et d'élution est de 16 cm/h. La chromatographie est effectuée à 20° C.

L'éluat est récupéré, puis dialysé, de préférence dans un tampon isotonique. En suivant le déroulement de la chromatographie, on constate que l'on peut charger au moins 0,5 g de β2'-glycoprotéine I par litre de lit de chromatographie.

On a analysé par électrophorèse dénaturante SDS PAGE l'éluat obtenu du gel d'hydroxyapatite (BIO-GEL HTP). On a constaté la présente d'une seule bande correspondant à la présence d'une protéine ayant un poids moléculaire de 50 000 ± 3 000 et ce, après ou sans réduction par le 2-mercaptoéthanol. La pureté de cette préparation a été testée en chromatographie dite "HPLC" sur "FRACTOGEL TSK 3000 SW" (appareil Tosho) et l'on constate également la présence d'un seul pic protéique.

La glycoprotéine ainsi isolée a les caractéristiques de compositions et séquences indiquées précédemment dans la présente description qui montrent que c'est une β2-glycoprotéine I allotype : la β2'-glycoprotéine I.

Les exemples donnés ci-après montrent la stabilisation de l'albumine par la composition contenant la β2'-glycoprotéine I.

### EXEMPLE 1

On prépare deux solutions d'albumine, A et B, de même concentration protéique (90 mg/ml), de même pH (7,1), et de même force ionique. La solution A est une solution d'albumine obtenue selon le procédé décrit ci-dessus mais n'ayant pas subi de traitement par chromatographie et la solution B est la solution d'albumine purifiée obtenue après chromatographie par affinité sur un gel de dextran sulfate pour enlever notamment la composition protéinique retenue sur la colonne de chromatographie. Ces deux solutions d'albumine A et B sont incubées à 60°C. A chaque heure d'incubation, et ceci pendant une durée de 5 heures, la turbidité de chaque solution est mesurée à l'aide d'un tubidimètre RATIO.XR.(HACH 43900).

La courbe 1 de turbidité en fonction du temps montre une turbidité, due à la chaleur, plus grande pour la solution d'albumine B que pour la solution d'albumine A, ce qui montre que l'on a enlevé de l'albumine A, grâce aux étapes chromatographiques, un inhibiteur de la dénaturation protéique due à la chaleur.

### EXEMPLE 2

A la solution d'albumine B, décrite dans l'exemple précédent, ayant une concentration protéique de 70 mg/ml, sont rajoutées des quantités croissantes d'éluat de la chromatographie d'affinité sur du dextran sulfate constituant la composition protéinique. La courbe 2 montre que lorsque la concentration de cet éluat augmente, la turbidité de l'albumine après 5 heures de chauffage à 60°C diminue. Cette courbe démontre d'une part que la composition protéinique agit comme inhibiteur de la tyndallisation et d'autre part que cette inhibition est dose dépendante.

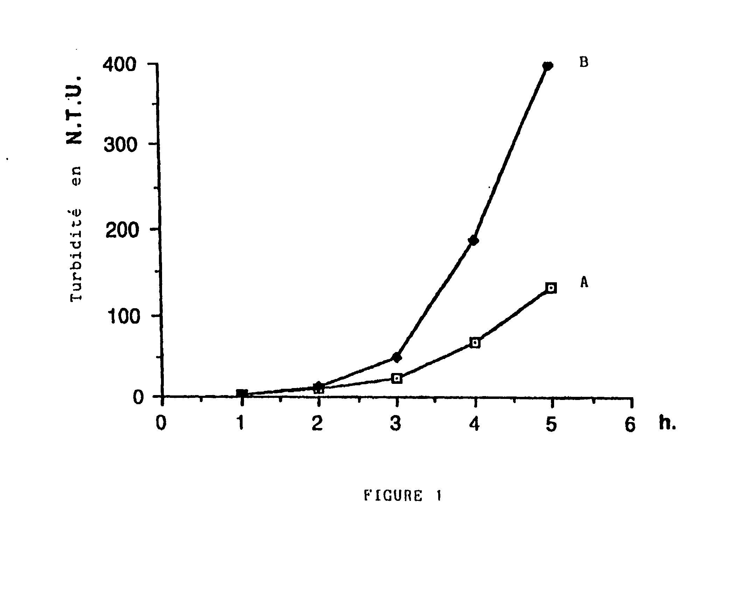

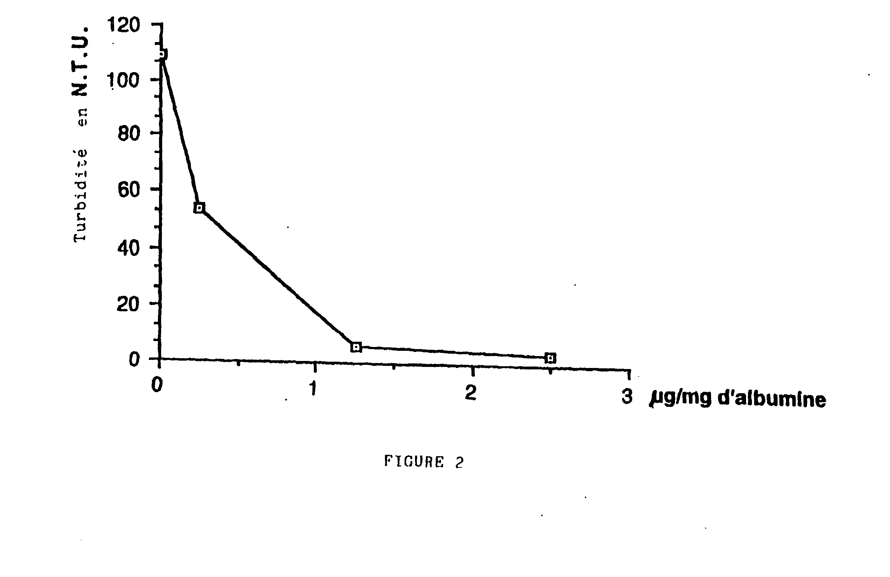

## Revendications

1. Procédé d'obtention d'une composition aqueuse protéinique à partir du plasma sanguin humain dans lequel on soumet le plasma à des traitements successifs par action de la température et par action d'un agent de précipitation pour aboutir, après séparation au moins partielle des facteurs VIII et IX et des gamma globulines à des solutions d'albumine contenant l'agent de précipitation, puis on sépare l'agent de précipitation des solutions obtenues pour obtenir une solution aqueuse brute d'albumine et on soumet ladite solution aqueuse brute d'albumine à au moins une chromatographie en phase liquide pour retenir au moins une partie des protéines secondaires autres que l'albumine, ladite chromatographie en phase liquide comportant une chromatographie d'affinité sur un support particulaire constitué de particules neutres chargées d'au moins un composé comportant des groupes sulfates, **caractérisé par le fait qu'**après passage de la solution d'albumine, on soumet le support particulaire de chromatographie d'affinité précité à une élution et que l'on recueille en élution la composition protéinique désirée.

2. Procédé selon la revendication 1, **caractérisée par le fait que** l'élution est effectuée par passage d'une solution saline en augmentant la force ionique.

3. Procédé selon la revendication 2, **caractérisé par le fait que** l'opération d'élution est réalisée au moyen d'une solution de NaCl.

4. Procédé selon l'une des revendications 2 ou 3, **caractérisé par le fait que** l'opération d'élution est précédée d'au moins une opération de lavage.

5. Procédé selon la revendication 4, **caractérisé par le fait que** le lavage est effectué à l'aide d'un tampon salin voisin de l'isotonicité dans des proportions donnant un pH de 7,00 ± 0,05.

6. Procédé selon l'une des revendications 4 ou 5, **caractérisé par le fait que** le lavage est maintenu tant que la densité optique de l'effluent est supérieure à une valeur prédéterminée.

7. Composition aqueuse protéinique obtenue par le procédé selon l'une des revendications 1 à 6.

8. Glycoprotéine (appelée β2'-glycoprotéine I) contenue dans la composition aqueuse protéinique selon la revendication 7 ayant un poids moléculaire de 50 000 ± 3 000 daltons après ou sans réduction par le 2-mercaptoéthanol dans laquelle les 20 premiers acides aminés de la région N-terminale sont les suivants :
Gly-Arg-Thr-Cys-Pro-Lys-Pro-Asp-Asp-Leu-Pro-Phe-Ser-Thr-Val-Val-Pro-Leu-Lys-Thr- et la séquence des acides aminés 315 à 321 est Phe-Trp-Lys-Ser-Asp-Ala-Ser.

9. Composition selon la revendication 7, **caractérisée par le fait qu'**elle contient de 5 à 100 % en poids de la glycoprotéine de la revendication 8 par rapport au contenu protéinique total.

10. Procédé de séparation de la glycoprotéine selon la revendication 8 à partir de la composition obtenue, selon l'une des revendications 1 à 6, par élution de la (des) colonne(s) de chromatographie d'affinité, **caractérisé par le fait que** la composition est traitée sur gel de dextran sulfate par une solution de NaCl au moins égale à 0,3 M; l'éluat obtenu est dilué dans un tampon salin de façon à abaisser sa force ionique au moins de 0,2 ; la solution obtenue est soumise à une chromatographie sur gel phosphatique, puis la glycoprotéine fixée sur le gel phosphatique est éluée en augmentant la force ionique avec un tampon salin de force ionique supérieure à 0,4.

11. Procédé selon la revendication 10, **caractérisé par le fait que** le débit de la chromatographie sur gel phosphaté est compris entre 10 et 20 cm/heure, la température étant comprise entre 0° C et 40° C.

12. Agent stabilisant l'albumine lors de sa tyndallisation, **caractérisé par le fait qu'**il contient la composition protéinique selon la revendication 7 ou la glycoprotéine selon la revendication 8.

13. Agent permettant la détection et/ou le dosage d'anticorps, de sujets humains infectés par HBV, HIV, du syndrome de Gougerot-Sjögren ou de myélomes en ELISA ou Immuno-empreinte **caractérisé par le fait qu'**il contient la composition protéinique selon la revendication 7 ou la glycoprotéine selon la revendication 8.

## Claims

1. Method for obtaining an aqueous protein composition from human blood plasma, in which the plasma is subjected to successive treatments by the action of temperature and by the action of a precipitation agent in order to lead, after at least partial separation of the factors VIII and IX and of the gamma globulins, to albumin solutions containing the precipitation agent, followed by separation of the precipitation agent from the solutions obtained in order to obtain a crude aqueous albumin solution, and the said crude aqueous albumin solution is subjected to at least one liquid phase chromatography in order to retain at least some of the secondary proteins other than the albumin, the said liquid phase chromatography including an affinity chromatography on a particulate support consisting of charged neutral particles of at least one compound containing sulfate groups, **characterized in that**, after passage of the albumin solution, the particulate support of the abovementioned affinity chromatography is subjected to an elution and **in that** the desired protein composition is collected on elution.

2. Method according to Claim 1, **characterized in that** the elution is carried out by passage of a saline solution, increasing the ionic strength.

3. Method according to Claim 2, **characterized in that** the elution operation is performed using an NaCl solution.

4. Method according to either of Claims 2 and 3, **characterized in that** the elution operation is preceded by at least one washing operation.

5. Method according to Claim 4, **characterized in that** the washing is carried out using a saline buffer close to isotonicity, in proportions giving a pH of 7.00 ± 0.05.

6. Method according to either of Claims 4 and 5, **characterized in that** the washing operation is continued for as long as the optical density of the effluent is greater than a predetermined value.

7. Aqueous protein composition obtained by the method according to one of Claims 1 to 6.

8. Glycoprotein (called β2'-glycoprotein I) contained in the aqueous protein composition according to Claim 7, having a molecular weight of 50,000 ± 3000 daltons after or without reduction by 2-mercaptoethanol, in which the first 20 amino acids of the N-terminal region are as follows:
Gly-Arg-Thr-Cys-Pro-Lys-Pro-Asp-Asp-Leu-Pro-Phe-Ser-Thr-Val-Val-Pro-Leu-Lys-Thr- and the sequence of amino acids 315 to 321 is the Phe-Trp-Lys-Ser-Asp-Ala-Ser.

9. Composition according to Claim 7, **characterized in that** it contains from 5 to 100% by weight of the glycoprotein of Claim 8 relative to the total protein content.

10. Method for separating the glycoprotein according to Claim 8, starting with the composition obtained according to one of Claims 1 to 6, by elution of affinity chromatography column(s), **characterized in that** the composition is treated on dextran sulfate by an NaCl solution which is at least equal to 0.3 M ; the eluate obtained is diluted in a saline buffer so as to lower its ionic strength of at least 0.2 ; the solution obtained is subjected to a chromatography on phosphate gel and the glycoprotein bound to the phosphate gel is then eluted by increasing the ionic strength with a saline buffer of ionic strength greater than 0.4.

11. Method according to Claim 10, **characterized in that** the flow rate of the chromatography on phosphate gel is between 10 and 20 cm/hour, the temperature being between 0°C and 40°C.

12. Agent which stabilizes albumin during its tyndallization, **characterized in that** it contains the protein composition according to Claim 7 or the glycoprotein according to Claim 8.

13. Agent which makes it possible to detect and/or assay antibodies from humans infected with HBV, HIV, Gougerot-Sjögren syndrome or myelomas on ELISA or immuno-imprinting, **characterized in that** it contains the protein composition according to Claim 7 or the glycoprotein according to Claim 8.

## Patentansprüche

1. Verfahren zur Herstellung einer wässrigen Proteinzusammensetzung aus humanem Blutplasma, wobei man das Plasma durch Einwirkung von Temperatur und eines Fällungsagens aufeinander folgenden Behandlungen unterwirft, um nach wenigstens partieller Abtrennung der Faktoren VIII und IX und von Gammaglobulinen zu Fällungsagens enthaltenden Albuminlösungen zu gelangen, man dann aus den erhaltenen Lösungen das Fällungsagens abtrennt, um eine rohe wässrige Albuminlösung zu erhalten, und man die rohe wässrige Albuminlösung wenigstens einer Flüssigphasenchromatographie unterwirft, um wenigstens einen Teil der von Albumin verschiedenen Sekundärproteine zurückzuhalten, wobei die Flüssigphasenchromatographie wenigstens eine Affinitätschromatographie auf einem partikulären Träger, bestehend aus neutralen, mit wenigstens einer Sulfatgruppen aufweisenden Verbindung beladenen Partikeln, beinhaltet, **dadurch gekennzeichnet, dass** man nach Durchlauf der Albuminlösung den partikulären Träger der vorstehend genannten Affinitätschromatographie einer Elution unterwirft, und man die gewünschte Proteinzusammensetzung durch Eluieren gewinnt.

2. Verfahren nach Anspruch 1, **dadurch gekennzeichnet, dass** die Elution durch Durchlauf einer Salzlösung bei ansteigender Ionenstärke bewirkt wird.

3. Verfahren nach Anspruch 2, **dadurch gekennzeichnet, dass** der Elutionsvorgang mit Hilfe einer NaCl-Lösung realisiert wird.

4. Verfahren nach einem der Ansprüche 2 oder 3, **dadurch gekennzeichnet, dass** dem Elutionsvorgang wenigstens ein Waschvorgang vorhergeht.

5. Verfahren nach Anspruch 4, **dadurch gekennzeichnet, dass** das Waschen mit Hilfe eines nahezu isotonischen Salzpuffers mit einen pH-Wert von 7,00 ± 0,05 ergebenden Anteilen bewirkt wird.

6. Verfahren nach einem der Ansprüche 4 oder 5, **dadurch gekennzeichnet, dass** man solange wäscht, bis die optische Dichte des Eluats über einem vorgegebenen Wert liegt.

7. Wässrige Proteinzusammensetzung, erhältlich mit dem Verfahren nach einem der Ansprüche 1 bis 6.

8. Glycoprotein, β2'-Glycoprotein I genannt, das in der wässrigen Proteinzusammensetzung nach Anspruch 7 enthalten ist und ein Molekulargewicht von 50.000 ± 3.000 Dalton nach Reduktion oder ohne Reduktion mit 2-Mercaptoethanol aufweist, worin die 20 ersten Aminosäuren der N-terminalen Region die folgenden sind:
Gly-Arg-Thr-Cys-Pro-Lys-Pro-Asp-Asp-Leu-Pro-Phe-Ser-Thr-Val-Val-Pro-Leu-Lys-Thr-
und die Sequenz der Aminosäuren 315 bis 321
Phe-Trp-Lys-Ser-Asp-Ala-Ser
ist.

9. Zusammensetzung nach Anspruch 7, **dadurch gekennzeichnet, dass** sie 5 bis 100 Gew.% des Glycoproteins des Anspruchs 8 bezogen auf den Gesamtproteingehalt enthält.

10. Verfahren zur Abtrennung des Glycoproteins nach Anspruch 8 aus der Zusammensetzung, die durch Elution der Affinitätschromatographiesäule(n) nach einem der Ansprüche 1 bis 6 erhalten wurde, **dadurch gekennzeichnet, dass** die Zusammensetzung auf Dextransulfatgel mit einer wenigstens 0,3 M NaCl-Lösung behandelt wird; das erhaltene Eluat in einem Salzpuffer so verdünnt wird, dass seine Ionenstärke auf weniger als 0,2 sinkt; die erhaltene Lösung einer Chromatographie auf Phosphatgel unterzogen wird, und dann das auf dem Phosphatgel fixierte Glycoprotein eluiert wird, indem man die Ionenstärke mit einem Salzpuffer von mehr als 0,4 erhöht.

11. Verfahren nach Anspruch 10, **dadurch gekennzeichnet, dass** der Chromatographiefluss auf phosphatiertem Gel 10 bis 20 cm/Stunde beträgt, wobei die Temperatur 0°C bis 40°C beträgt.

12. Agens, das Albumin bei seiner Tyndallisierung stabilisiert, **dadurch gekennzeichnet, dass** es die Proteinzusammensetzung nach Anspruch 7 oder das Glycoprotein nach Anspruch 8 enthält.

13. Agens, mit dem sich Antikörper aus humanen Wesen, die mit HBV, HIV, mit dem Gougerot-Sjögren-Syndrom oder Myelomen infiziert sind, im ELISA oder Immunabdruck nachweisen und/oder messen lassen, **dadurch gekennzeichnet, dass** es die Proteinzusammensetzung nach Anspruch 7 oder das Glycoprotein nach Anspruch 8 enthält.
